# EUROPEAN PATENT APPLICATION

(11) **EP 2 431 387 A1**
(43) Date of publication of application: **21.03.2012**
(21) Application number: 10305987.9
(22) Date of filing: 15.09.2010
(51) Int. Cl.: C07K 14/705, C07K 14/72, C12N 15/81, C12P 21/02, C07K 1/14, G01N 33/68

(54) **A raft deficient yeast expression system for the production of recombinant membrane proteins**

(71) Applicant: Centre National de la Recherche Scientifique (C.N.R.S), 75016 Paris (FR); Université de Strasbourg, 67000 Strasbourg (FR)
(72) Inventor: Wagner, Renaud, 67640 Lipsheim (FR); Alkhalfioui, Fatima, 67100 Strasbourg (FR)
(74) Representative: Blot, Philippe Robert Emile

(57) **Abstract**

The present invention relates to a raft deficient yeast expression system for the production of heterologous membrane proteins, preferably proteins that are naturally raft-associated, more preferably G-protein coupled receptors. This cell based expression system in which recombinant membrane proteins are functionally expressed in a homogeneous form and efficiently extracted from membranes by detergents involves genetically modified yeasts wherein at least one gene controlling raft formation, constitution and/or integrity is modified. It also relates to a method for the preparation of said yeasts. It finally relates to the utilization of said yeasts for isolation and purification of said proteins, for their biochemical, biophysical and functional characterization such as for example structure determination and ligand screening.

## Description

The present invention relates to a method for producing a recombinant membrane protein, preferably a raft-associated recombinant protein, more preferably a recombinant G-protein coupled receptor protein. The present invention also relates to a method for crystallizing a recombinant membrane protein and to a method for screening for ligands of recombinant membrane proteins.

G-protein coupled receptors (GPCRs), including more than 900 members, represent the largest family of membrane proteins. They sense a large panel of extracellular stimuli (ions, biogenic amines, purines, lipids, peptides, proteins, odorants and tastants) that they transduce into the cells, participating in the regulation of major (patho)physiological processes such as neurotransmission, proliferation, differentiation, chemotaxis or inflammation.

With already about 30 % of the marketed drugs acting on only 20 GPCRs, these key signalling proteins represent ideal therapeutic targets for a number of disorders and diseases and still constitute the central core for drug discovery in pharmaceutical industry programmes. High-resolution 3D structures of GPCRs are notably highly desired, not only for a fine understanding of their functioning at the atomic level, but also for holding accurate models that can be exploited in structure-based drug design approaches (drug screening and diagnostic applications). Along with the exploitation of GPCRs structures, the availability of milligram quantities of active purified receptors opens up new routes to decipher unprecedented activities of orphan receptors, to identify novel interacting partners or to determine receptor behaviour in different model environments (Alkhalfioui et al. 2009 Curr Opin Pharmacol. 9(5):629-35), and brings the proteic material suitable for a large panel of downstream applications such as antibody production, protein array construction, functional assays, ligand screening and biophysical characterization among others. Producing and recovering workable amounts of pure, homogeneous, stable and active GPCRs for these different applications is however extremely challenging so that reliable and robust tools and methods are still highly wanted.

Apart from the very singular exception of rhodopsin, GPCRs are naturally poorly abundant in tissues so that heterologous expression systems are required. Among the most widespread, popular, effective and inexpensive microorganisms developed for heterologous expression, the yeast *Pichia pastoris* has become a system of choice not only for the production of cytosoluble and industrially relevant proteins, but also for a growing panel of eukaryotic membrane proteins. Up to now indeed, more than 150 different representative membrane proteins including a number of GPCRs have been successfully expressed in *P. pastoris. Moreover, a dozen of them could be produced and purified at a sufficiently high level of quantity and quality* to be cristallyzed and their 3D structure solved, making *P. pastoris* one of the most performant heterologous expression system for the structural studies of eukaryotic membrane proteins. Several membrane proteins however, including GPCRs, still fail to be extracted and purified from this expression system to levels compatible with structure determination.

Several lines of evidence indicate that the lipids and the proteins in biological plasma membranes are not homogeneously and randomly distributed. Instead, plasma membranes are compartimentalized into microdomains ('rafts') with compositions and physical properties that differ from the rest of the plasma membrane. These membrane microdomains are enriched with sterols, glycolipids, sphingolipids and specific proteins. The hydrophobic chains of the lipids contained in the rafts are more saturated and tightly packed than the surrounding bilayer. Several microdomains housing specific lipids and proteins may coexist in the membrane, such as lipid rafts or caveolae. Altogether, these specialized microdomains are believed to play important roles in many cellular processes, particularly in signal transduction, lipid/protein sorting and trafficking. The existence of microdomains in the membrane has been suggested based on the biochemical observation that a subset of membrane components is resistant to solubilization after extraction with non-ionic detergents such as Triton-X100 or CHAPS at 4°C (Brown & Rose 1992 Cell. 68(3):533-44). When such detergents are added to membranes, the fluid membrane dissolves while the rafts remain intact. This resistance is in fact due to the tight and strong interaction between sterols, sphingolipids, glycolipids and proteins. Because of their composition and detergent resistance, these microdomains are also referred to as Detergent Resistant Membranes (DRMs), Detergent Insoluble Glycolipid enriched complexes (DIGs), Low Density Triton X-1 00 insoluble (LDT1 ) membrane domains.

In yeast membranes, microdomains are dynamic platforms containing high levels of ergosterol and sphingolipids which segregate and concentrate subsets of membrane proteins. The presence or absence of ergosterol in lipid raft has been shown to influence the distribution and the biological activity of the HUP1 hexose-proton symporter raft associated protein (Grossmann et al. 2006 Eukaryotic Cell 5(6):945-53). Evidence has been accumulating over the past few years that a range of GPCRs is targeted either constitutively or after agonist stimulation to rafts both in natural sources and in heterologous expression systems.

In heterologous expression systems, this particular localization of recombinant GPCRs in rafts has a critical impact on their extraction. This detergent-resistance has made the receptor difficult to purify in high quantities.

There is thus a need in the art for a method allowing the easy and efficient solubilization from the membranes, by detergents, of a high proportion of GPCRs to be purified (e.g. more than 50%). Ideally, this method should also allow the obtention of high amounts of homogeneous, active and stable solubilized receptors.

The present invention arises from the unexpected finding, by the inventors, that the use of an engineered yeast strain lacking membrane rafts dramatically enhanced the production and the extraction of GPCRs, allowing the recovery of importantly higher amounts of homogeneous, active and stable receptors for their subsequent utilization in various applications. Particularly, the inventors have demonstrated that a mutation in an ergosterol biosynthesis gene so that the yeast failed to synthesize ergosterol positively affected the functionality, homogeneity, stability and solubilizability of a series of GPCRs. Such a strain is thus a useful tool facilitating the subsequent biochemical and structural studies on GPCRs as well as other membrane proteins that are associated to rafts. Their results provide the basis both for methods for screening for binding compounds and for a method for crystallizing a membrane protein associated to rafts.

### Raft lipid deficient yeast strains according to the invention

As intended herein, a "yeast" may be any kind of yeast. For example, the yeast can be chosen from the genera *Saccharomyces, Kluyveromyces, Candida, Schizosaccharomyces, Pichia, Yarrowia, Hansenula, Phaffia, Arxula, Debaryomyces, Issatchenkia orientalis* and *Schwanniomyces.* Preferably, the yeast genus is *Pichia,* more preferably the species is *Pichia pastoris,* even more preferably, the strain is the GS115 strain of *Pichia pastoris* (De Schutter et al. 2009 Nat Biotechnol. 27(6):561-6).

According to the invention, the yeasts are characterized in that they do not synthesize one or more raft lipids. Preferably, the raft lipid which is not synthesized by said yeast is ergosterol or a sphingolipid.

As intended herein, the term "rafts" refers to specialized micro-domains containing high levels of cholesterol, ergosterol and/or sphingolipids which are generally present in yeast or animal cells. Rafts segregate and concentrate subsets of membrane proteins, herein referred as "raft associated proteins". According to the invention, the rafts are preferably micro-domains which resist to detergent treatment, such as Triton X-100 at low temperature (4°C). Example of micro-domains are lipid rafts, caveolae, Detergent Resistant Membranes (DRMs), Detergent Insoluble Glycolipid enriched complexes (DIGs), Low Density Triton X-100 insoluble (LDT1) membrane domains, Membrane Compartment of Can1 (MCC), Membrane Compartment of Pma1 (MCP) among others.

According to the invention, a "raft lipid deficient yeast strain" or a "yeast strain which is deficient in a raft lipid biosynthesis" is a yeast strain which does not synthesize or synthesize little of one or more raft lipids. The expression "little raft lipid biosynthesis" is intended to mean the synthesis of a quantity of one or more raft lipid which is neither sufficient to stabilize membrane rafts nor to recruit proteins into the rafts. For example, when the raft lipid is ergosterol, an ergosterol deficient yeast strain that produces "little ergosterol" may synthesize less than 10%, less than 20%, less than 30% or less than 50% of the amount of ergosterol produced by a strain which is not deficient.

Such raft lipid deficient yeast strain do preferably not contain any rafts.

In a preferred embodiment according to the invention, the yeast strain is an ergosterol deficient yeast strain. Ergosterol (ergosta-5,7,22-trien-3ß-ol) is the major product of sterol biosynthesis and the major membrane/raft lipid in yeast, and is a biological precursor (a provitamin) of vitamin D2. Preferentially, the "ergosterol deficient yeast strain" does not synthesize any ergosterol at all.

Alternatively, the yeast strain according to the invention is a sphingolipid deficient yeast strain. The sphingolipids are a class of lipids derived from the aliphatic amino alcohol sphingosine. A sphingolipid according to the invention may, for example, be a sphingomyelin, a glycosphingolipid, or a gangliosid.

Such raft lipid deficient strains can be obtained by methods well-known to the skilled in the art, e.g. by random or directed mutagenesis (for example in modifying gene(s) which may intervene in the biosynthesis of raft lipid), or by any treatment of the yeasts by chemical or biological compounds that are inhibiting the biosynthesis of raft lipid. Such chemical compounds may be for instance ergosterol synthesis inhibitors like azoles (e.g. ketoconazole, fluoconazole or itraconazole), allylamines (e.g. terbinafine, butenafine), or morpholines (e.g. amorolfine)..

According to one way of carrying out the invention, the raft lipid deficient strain may for example be obtained by random mutagenesis, in particular by exposing the yeasts to a treatment by means of mutagenic agents. The mutagenic agent may, for example, be a physical agent such as X-rays, gamma-rays and ultra-violet rays or a chemical agent such as alkylating agents, dialkylating agents and intercalating agents.

A protocol for obtaining an ergosterol deficient strain by random mutagenesis is notably described in the example.

According to a further way of carrying out the invention, the "ergosterol deficient strain" may be obtained by directed mutagenesis. The target for mutagenesis may be any gene involved in ergosterol biosynthesis through inactivation of a gene playing a role in ergosterol biosynthesis. Preferentially, the gene to be mutated is not an essential gene for yeast. In particular, it may be one of the following: *erg1, erg2, erg3, erg4, erg5, erg6, erg7, erg8, erg9, erg10, erg11, erg12, erg13, erg20, erg24, erg 25, hmg1, hmg2, idi1, mvd1, sur2, elo3, lac1, lag1, lcb2, lcb3, lcb4, lcb5, tsc10, ydc1 and ypc1.* More preferentially, the gene is *erg2, erg3,* erg4, *erg5* and *erg6.* Even more preferably the gene is *erg 6. erg6* encods the Delta(24)-sterol C-methyltransferase enzyme that converts zymosterol to fecosterol in the ergosterol biosynthetic pathway by methylating position C-24 and is preferably represented by SEQ ID NO: 1, the encoded ERG6 protein can notably be represented by SEQ ID NO:2. A protocol for obtaining an ergosterol deficient strain by mutagenesis of *erg6,* is notably described in the example.

Methods for carrying out directed mutagenesis in yeast are well known by the skilled person and are described, for example, in Rothstein 1991 Methods Enzymol. 194:281-301; Wach et al. 1994 Yeast 10:1793-1808 and also in Goldener et al. 1996 Nucleic Acids Res. 24:2519-254.

An inactivated gene is understood to mean a gene which has been partially or totally incapable of encoding its natural protein. The incapacity of the said genes to encode their natural proteins may manifest by the production of an inactive protein, by the absence of production or by the production of a mutant having a modified activity.

This gene inactivation may, for example be induced by one or more mutations or by deletion. Mutation should be understood to mean, in particular, any substitution, deletion and/or addition of one or more bases in the nucleotide sequence of the considered gene.

The substitution may in particular involve the introduction of a non-sens mutation in the coding sequence.

Deletion is understood to mean any interruption of the gene as well as partial or total suppression of the gene considered. The said genetic modification(s) may also be obtained by gene disruption or gene replacement (double homologous recombination). In this case, the coding sequence will be preferably partly or totally perturbed so as to allow the introduction, by homologous recombination, of a foreign nucleotide sequence in the wild type genomic sequence. The foreign nucleotide sequence may be:
- a non-functional/mutant sequence,
- a gene involved in a biosynthetic pathway in yeast,
- a selective gene that allows the expression of a selection marker.

The selective gene may be an antibiotic resistance gene, such as a geneticin or zeocin resistance gene. Alternatively, the selective gene may be the wild-type gene involved in a biosynthetic pathway in yeast when the yeast strain itself carries a mutated and inactive allele of said gene (for example, URA3, TRP1, LEU2 or HIS3).

These mutations may be localized in the coding sequence or outside the coding sequence, for example in the regions responsible for the expression and/or the transcriptional regulation of the said gene(s), e.g. the gene promoter region.

Mutant strains which are deficient in ergosterol biosynthesis can for example be identified by the following methods which are well-known by the skilled person. For example, a yeast strain which does not produce ergosterol is identifiable by its resistance to nystatin. A way to perform a resistance test to nystatin is notably described in the example. Nystatin is an antibiotic which specifically binds to ergosterol and induces the formation of pores, which leads to yeast cell lysis. Alternatively, once the yeast membranes are separated, their sterol composition can be measured by HPLC and analysis of the chromatographic elution patterns of the sterol species, as described in the example.

### Membrane proteins that can be produced according to the invention

Membrane proteins are localized in the cell membranes. Such biological membranes include the plasma membrane that is separating the interior of a cell from the outside, as well as membranes compartmenting intracellular organelles such as the endoplasmic reticulum or the Golgi apparatus for instance. The cell membrane according to the invention is preferably the plasma membrane. The recombinant membrane protein in accordance with the invention may be homologously or heterologously expressed in yeast. Preferentially, it is a protein of microbial, animal or plant origin, more preferentially, of human origin.

In accordance with the invention, the membrane protein is preferentially a raft-associated protein.

The "raft-associated protein" according to the invention is a protein which is known to associate with raft in natural sources, and/or in raft containing expression systems (such as a yeast). This association can be permanent or become associated following stimulation, for example by an external signal received by the cell.

The raft-associated proteins are known to be resistant to solubilization by detergents. Thus, according to the invention, one method to characterize proteins as raft-associated proteins is to test their resistance to detergents. For example, the membranes of animal or yeast cells can be solubilized in the presence of detergents such as Triton-X100, CHAPS and the quantity of raft-associated proteins can be measured in the soluble fraction of the extract, as described in particular, in the example. A weak solubility is indicative of the fact that the proteins are raft-associated. The term "weak solubility" is intended to mean that the quantity of solubilized proteins is less than 50%, 60% or less than 70% of the total amount of proteins present in the membranes before solubilization. In some instances, the quantity of solubilized proteins may be less than 40%, 30% or, preferentially, less than 20% of the total amount of proteins present in the membranes before solubilization.

Alternatively, the raft-associated proteins can equally be identified by isolating the raft domains on a density gradient, as described for example in Bagnat et al. (2000 PNAS 97:3254-3259), or by using specific imaging techniques such as the two-photon microscopy, as described for example in Gauss et al. (2003 PNAS 100:15554-15559).

Many proteins are known by the skilled person as being associated to rafts. These proteins may contain transmembrane domains. Alternatively, they are associated to rafts in different ways, by means of glycosylphosphatidyinositol (GPI) anchors, via myristate/palmitate motives or dual palmitate motives. Preferentially, the raft-associated proteins according to the invention are transmembrane proteins. As examples of raft-associated proteins, one finds B or T- cell receptors, FcεRI, members of the rc-family of kinases, some SNARE proteins, several phosphatases and transporters as well as G protein-coupled receptors (GPCRs).

According to the invention, the raft-associated proteins preferably are G protein-coupled receptors (GPCRs). As intended herein, the "G protein-coupled receptors", also known as seven-transmembrane domain receptors, 7TM receptors, heptahelical receptors, serpentine receptors, and G protein-linked receptors (GPLR), comprise a large family of transmembrane receptors that bind molecules outside the cell and subsequently activate signal transduction pathways inside the cells and, ultimately, cellular responses. The "G protein-coupled receptors" generally share a highly conserved structural signature of seven transmembrane segments which display a high degree of hydrophobicity, three extracellular loops [EL1-3], three intracellular loops [IL1-3] as well as an extracellular N-terminus and an intracellular C-terminus.

The GPCRs according to the invention may belong to either of the 6 classes of GPCRs listed below, based on sequence homology and functional similarity,
Class A (Rhodopsin-like)
Class B (Secretin receptor family)
Class C (Metabotropic glutamate/pheromone)
Class D (Fungal mating pheromone receptors)
Class E (Cyclic AMP receptors)
Class F (Frizzled/Smoothened)

For example, the GPCR can be a Muscarinic M1 receptor, a Histamine receptor, a 5-HT GPCR, Cannabinoid, a Class A hormone protein, a Galanin, a Melanocortin, a Neuropeptide Y receptor, a Neurotensin, a Somatostatin, a Vasopressin like receptor, a Prostanoid receptor, a ACTH releasing factor receptor, a GABA B receptor, a Metabotropic glutamate receptor, a adrenergic receptor (such as ADRA1A, ADRA2B), a dopamine receptor (such as DRD2) or a melatonin receptor (such as MTR1 A).

### Method for producing a recombinant membrane protein

The present invention relates to a method for producing a recombinant membrane protein in yeast which comprises:
- providing a yeast strain deficient in raft lipid biosynthesis, preferably deficient in ergosterol biosynthesis,
- transforming said yeast strain with a vector comprising a sequence coding for said recombinant membrane protein, and
- growing said yeast strain under conditions allowing expression of said recombinant membrane protein.

Preferably, the recombinant membrane protein is a recombinant raft associated protein, more preferably a recombinant GPCR protein.

The term "transformation" according to the invention means the introduction of a DNA sequence into the yeast cell, so that the yeast cell will express the introduced gene or sequence to produce a desired protein coded by the introduced gene or sequence. The introduced gene may include regulatory or control sequences, such as start or stop signals, secretion signal or promoter, or other sequences used by the yeast genetic machinery. Preferentially, it is the gene coding sequence which is introduced in the host cell. The coding sequence (also called CDS for coding DNA sequence) is the portion of the gene which is translated into a protein.

Transformation methods are well-known by the skilled person. Preferentially, transformation is carried out by electroporation, notably by electroporation with linearized DNA. Alternatively, transformation may be achieved on spheroblasts in the presence of CaCl₂, the spheroblasts being obtained by lysis of the cell wall with various enzymes such as a lyticase.

The gene or gene sequence is preferably introduced into the yeast by a vector. According to the invention, the term "vector" typically comprises the DNA of a transmissible agent, into which foreign DNA is inserted. A common way to insert one segment of DNA into another segment of DNA involves the use of enzymes called restriction enzymes that cleave DNA at specific sites (specific groups of nucleotides), called restriction sites. A "cassette" refers to a DNA coding sequence or segment of DNA that codes for an expression product, that can be inserted into a vector at defined restriction sites. The cassette restriction sites are designed to ensure insertion of the cassette in the proper reading frame. Generally, foreign DNA may be inserted at one or more restriction sites in the vector DNA, and is, then, carried into the host cell along with the transmissible vector DNA.

Preferably, the vector is a " lap smid", which generally is a self-contained molecule of double-stranded DNA, which can readily accept additional DNA and which can readily be introduced into a suitable yeast cell.

The terms "express" and "expression" mean allowing or causing the information in a gene or DNA sequence to become manifest, for example, it means producing a protein by activating the cellular functions involved in transcription and translation of the gene or DNA sequence.

According to the invention, an "expression vector" allows the expression of the gene encoding the membrane protein under the control of a promoter recognized by the yeast cell. The expression vector according to the invention may be an autonomously replicating vector such as a plasmid or an artificial chromosome. It may be an integrative vector which may be inserted in one or more of the yeast chromosomes. It may be, for example, the pPIC9K vector or an engineered version as notably described in André et al. (2006 Protein Science 15 :1115-1126).

According to the invention, the promoter preferentially is a yeast promoter, more preferentially a strong yeast promoter. A strong yeast promoter is a promoter which enables a higher level of transcription of the gene which is under its control (generally directly downstream of it) than the average level of transcription of yeast genes. Strong yeast promoters are described for example in Velculescu et al. (1997 Cell 88: 243-251). Examples of strong yeast promoters are the natural promoters of the PGK1, ADH1, nmt1, PRB1, PHO5, PDR5, HXT2, PMA1, CYC1, GAL1, AOX1, FLD1, TEF1, MOX1 and GAP1 genes. The promoter may be constitutive or inducible. According to the invention, the promoter preferentially is a strong and inducible promoter such as the methanol inducible AOX1 promoter.

The vector may also comprise a tag coding sequence. Preferentially, this sequence is positioned directly downstream of the gene encoding the recombinant protein in such a way that the recombinant protein which is expressed carries the tag sequence. For example, the tag sequence according to the invention may be a flag tag, a histidine tag, a biotinylation tag or an EGFP tag.

The yeast strain transformed with the expression vector comprising the sequence coding for a recombinant membrane protein is grown under conditions allowing the expression of said protein. These conditions can be easily determined by the person skilled in the art and they depend on the yeast strain, the vector and the promoter which are used. For example, a yeast strain, such as a *P. pastoris* strain transformed with an expression vector bearing the AOX1 promoter, may be grown in a BMGY culture medium, at 30°C, up to an optical density of 2 to 6 and, then, be resuspended at an optical density of 1 in an induction medium comprising 0.5% methanol instead of glycerol as a carbon source (BMMY) and grown for 18 hours.

Methods for increasing recombinant GPCR protein production which may be used in accordance with the invention are described more particularly in André et al. (2006 Protein Science 15 :1115-1126).

The present invention also relates to the use of a yeast strain deficient in a raft lipid biosynthesis, more preferably in ergosterol biosynthesis for the production of a recombinant membrane protein, preferably a Raft recombinant associated protein, even more preferably a recombinant GPCR protein.

The present invention also relates to a yeast strain deficient in a raft lipid biosynthesis, more preferably in ergosterol biosynthesis transformed with an expression vector comprising the sequence coding for a recombinant membrane protein, preferably a Raft recombinant associated protein, even more preferably a recombinant GPCR protein.

In a preferred embodiment, the yeast strain according to the invention carries a mutation in the *erg6* gene as represented by SEQ ID NO: 3, the encoded truncated protein being represented by SEQ ID NO: 4.

### Extraction and purification

The recombinant membrane proteins, preferably the recombinant raft-associated proteins, even more preferably the recombinant GPCR proteins, produced by a method according to the invention, can be extracted from the yeast culture and optionally purified.

The cells of the yeast culture used as starting material for producing the recombinant proteins is called "yeast whole cells".

Protein extraction may comprise several steps including the step of separating the cell membrane.

The cell membrane can be obtained by lysis of the yeast whole cells followed by centrifugation to eliminate the cells which have not been lysed and the cellular debris. For example, cell lysis can be accomplished by harsh vortexing using glass beads or dedicated cell disrupters. Centrifugation can be carried out at low speed, for example, 4000 rpm. A method for lysing and separating the unlysed cells and the cell debris is notably described in the example.

The cellular membranes can be obtained, for example, by ultracentrifugation as notably described in the example. In the present description, they are also named the "cell membrane fraction".

The membrane proteins can be extracted from the cell membrane fraction. The extraction procedure preferentially comprises the solubilization of this fraction by a detergent. Preferentially, the detergent is triton-X100, DDM/CHS or CHAPS/CHS, more preferentially, triton-X100. Preferentially the final concentration in detergent is less than 2%, preferably less than 1% and alternatively above the critical micelle concentration (cmc).

After solubilization, the insoluble fraction can be separated from the solubilized membrane proteins for example by ultracentrifugation as notably described in the example (Materials and methods). A method for solubilizing the membranes and separating the insoluble fraction is notably described in the example. A "solubilized membrane protein fraction" is, thus, obtained.

The recombinant membrane proteins, in particular, those which are raft-associated, can be purified from the solubilized membrane protein fraction. This purification can be achieved in particular with the help of the tags carried by the recombinant membrane protein. Such purification methods are well-known by the skilled person and they depend on the kind of tags which have been used. For example, the recombinant membrane protein may be purified by affinity chromatography with the help of antibodies which recognize the tag. Alternatively, when the tag is a histidine tag, purification can be achieved by an immobilized metal affinity chromatography (IMAC) procedure. When the tag is a biotinylation tag, purification can be achieved on the basis of affinity for streptavidin. A "purified recombinant membrane protein fraction" is, thus, obtained.

In a preferred embodiment, the method according to the invention allows extracting and/or purifying at least 40%, 50%, 60%, more preferably 70%, 80% or more of the recombinant membrane proteins.

The proteins thus obtained are suitable for a large panel of downstream applications such as, for example, antibody production, protein array construction, functional assay, ligand screening and biophysical characterization among others.

### Recombinant membrane protein activity

The conformation of a protein depends on the folding of the polypeptide chain in space. It is also known as its tridimensional structure. The conformation of a protein is intimately linked to its function, a denatured protein being inactive.

According to the invention, the expression « active conformation » refers to the conformation of a recombinant membrane protein which is not denatured. This conformation enables them to bind their ligands and/or to accomplish their biological activity. The expression « active conformation may also be replaced by « ligand binding competent conformation ».

In a preferred embodiment, the recombinant membrane protein is a recombinant raft associated protein more preferably a recombinant GPCR protein. Preferably, when the recombinant membrane protein is a recombinant receptor, for example a recombinant GPCR protein, the conformation of said recombinant receptor may be deduced from its capacity to bind its ligand.

Ligands are known for a high number of GPCR proteins. They can be found for example in the database GLIDA (GPCR-Llgand DAtabase) (Yang et al. 2004 Genome Informatics 15: P057, Okuno et al. 2006 Acids Research 34: D673-D677). Alternatively, the ligand for a GPCR protein can be identified by a screening method according to the invention, as described below.

Methods for measuring the binding of a ligand to its receptor, preferably to a recombinant GPCR protein, are well known by the skilled person. These methods notably include saturation binding assays such as radioactive ligand binding assay (filtration assay, scintillation proximity assay), fluorescent and FRET based assay or luminescent oxygen channeling based assay, for example AlphascreenⓇ.

For example, a saturation binding assay can involve the addition of increasing amounts of the labeled ligand to a fraction containing the recombinant receptors. The amount of specifically bound ligand is, thereafter, determined. For example, if the ligand is radioactively labeled, the amount of specifically bound ligand may be ascertained by liquid scintillation counting. This measurement then allows the calculation of the dissociation constant at equilibrium (Kd) and an evaluation of the maximum number of binding sites (B max) of the ligand to its receptor. How to carry out such a method is notably described in the example. According to the invention these measures can be performed on various fractions comprising whole cell fraction, cell membrane fraction, solubilized membrane protein fraction or purified recombinant membrane protein fraction.

The total number of membrane proteins can be determined by methods well known in the art. For example, it can be determined in the cell membrane fraction or in the solubilized membrane protein fraction by a BCA assay with BSA used as standard.

The total number of recombinant membrane protein corresponds to the number of recombinant membrane protein produced in active and inactive conformation. It can be measured by techniques well known by the skilled person. For example, it can be determined in the purified recombinant membrane protein fraction by a BCA assay with BSA used as standard. Alternatively, signal intensities from a dot-blot immunodetection can be quantified to determine the relative amount of recombinant membrane proteins in parallel experiments.

According to another embodiment, the number of recombinant membrane proteins in an active conformation compared to the total number of recombinant membrane proteins is at least 10 % higher, preferably at least 50%, 100%, 200% or 300% higher, most preferably more than 300% higher in the yeast strain deficient in raft lipid biosynthesis than it would be in yeast strains synthesizing said raft lipid (e.g. ergosterol).

In a preferred embodiment, the recombinant membrane protein is a recombinant GPCR protein. The number of recombinant GPCR proteins in an active conformation is preferably estimated by the B max value, as notably described in the example.

According to the invention, the Kd values of recombinant GPCR protein produced by a yeast strain which is deficient in ergosterol biosynthesis and by a wild-type strain are similar.

### Method for screening for recombinant membrane protein binding compounds

One aspect of the invention pertains to methods for screening for a ligand of a recombinant membrane protein.

Thus the present invention relates to a method for screening for a ligand of a recombinant membrane protein which comprises the steps of:
i) providing a yeast strain deficient in raft lipid biosynthesis, more preferably deficient in ergosterol biosynthesis,
ii) transforming said yeast strain with an expression vector comprising a sequence coding for said recombinant membrane protein,
iii) growing said yeast strain under conditions allowing expression of said recombinant membrane protein,
iv) contacting at least one candidate ligand with whole cells of the yeast strain grown at step iii), with a cell membrane fraction obtained therefrom, with a solubilized membrane protein fraction obtained therefrom or with a purified recombinant membrane protein fraction obtained therefrom, and
v) determining whether said candidate compound binds to the recombinant membrane protein.

"Candidate ligand" is used herein to define a ligand which is potentially able to bind to the recombinant membrane protein.

The nature of the candidate ligand may vary: it can for example be a protein, a peptide, a small molecule, an organic compound, a lipid, a sugar or a nucleic acid. Preferentially, it is a protein or a peptide.

The candidate ligand may, for example, be a natural ligand of the recombinant membrane protein, an agonist, an antagonist thereto or an antibody directed against said recombinant membrane protein.

Methods for screening for a ligand of a recombinant membrane protein comprise the step of producing the recombinant membrane proteins according to the invention.

The candidate ligand can then be in contact with the whole yeast cell, with the cell membrane fraction, with the solubilized membrane protein fraction or with the purified recombinant membrane protein fraction obtained according to the invention.

The ability of the candidate ligand to bind to the recombinant membrane protein can be determined by methods known by the person skilled in the art, in particular the methods mentioned above which comprise saturation or competition binding assays such as radioactive ligand binding assay (filtration assay, scintillation proximity assay, dialysis assay, precipitation assay), immunological-based assay such as ELISA assay, SPR-based assay, fluorescent and FRET based assay and luminescent oxygen channeling based assay, for example AlphascreenⓇ.

These methods allow the determination of whether or not the candidate ligand binds to the recombinant membrane protein and, accordingly of whether or not it is a ligand for this protein.

### Method for cristallizing a recombinant membrane protein

Another aspect of the invention is directed to methods for crystallizing a recombinant membrane protein which comprise:
i) providing a yeast strain deficient in raft lipid biosynthesis, more preferably deficient in ergosterol biosynthesis,
ii) transforming said yeast strain with an expression vector comprising a sequence coding for said recombinant membrane protein,
iii) growing said yeast strain under conditions allowing expression of said recombinant membrane protein.
iv) extracting and purifying said recombinant membrane protein, and
v) crystallizing the recombinant membrane protein thus obtained.

The crystallization of the recombinant membrane protein can be achieved by various methods well known by the person skilled in the art, such as the Vapor Diffusion crystallization, the batch crystallization, the dialysis crystallization.

In a vapor diffusion experiment, small volumes of the precipitating agent and the protein can be mixed together and the drop equilibrated against a larger reservoir of solution containing the precipitating agent or another dehydrating agent.

In a batch crystallization experiment, the precipitant agent and the protein can be mixed directly under oil.

In a dialysis crystallization experiment, the protein can be equilibrated against a larger volume of precipitant agent through a dialysis membrane.

In a preferred embodiment, the recombinant membrane protein which is cristallized is in an active conformation.

In another preferred embodiment, the recombinant membrane protein has a known ligand and the method for cristallization according to the invention comprises the step of adding this ligand to the recombinant membrane protein before the step of crystallizing the protein.

Thus the present invention also relates to a method for crystallizing a membrane protein which comprises the step of stabilizing the recombinant membrane protein by adding a ligand of said recombinant protein before or during the step of extracting and purifying said membrane protein.

The ligand may be directly added to the whole yeast cells, to the cell membrane fraction, to the solubilized membrane protein fraction or to the purified recombinant membrane fraction obtained according to the invention. Preferentially, it is added to whole yeast cells or to the cell membrane fraction. This addition may stabilize the recombinant membrane protein in active form.

### Kit

The present invention also relates to a kit for producing a recombinant membrane protein comprising a yeast strain deficient in raft lipid biosynthesis, more preferably deficient in ergosterol biosynthesis, the appropriate buffers and reagents and the instructions for use.

The appropriate buffer and reagents can be any buffer or reagent suitable for producing a recombinant membrane protein, in particular buffer and reagent useful for transforming the yeast strain with an expression vector, and/or for allowing the expression of a recombinant protein and/or for extracting or purifying the recombinant protein produced.

The instructions for use may describe how to perform the method of production of a recombinant protein according to the invention.

### Brief description of the drawing

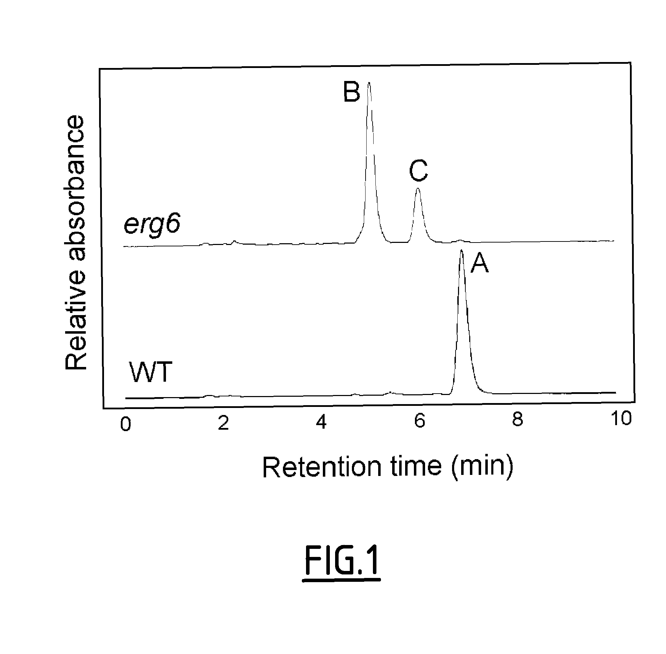

**FIG. 1**: Profiles of sterols extracted from membranes of wild type (WT) and *erg6* mutant cells *(erg6)* separated by HPLC chromatography and identified by GC-MS spectrometry. Peak A correspond to ergosterol; peak B correspond to cholesta-5,7,24-trien-3ß-ol; peak C correspond to cholesta-5,7,22,24-tetraen-3ß-ol

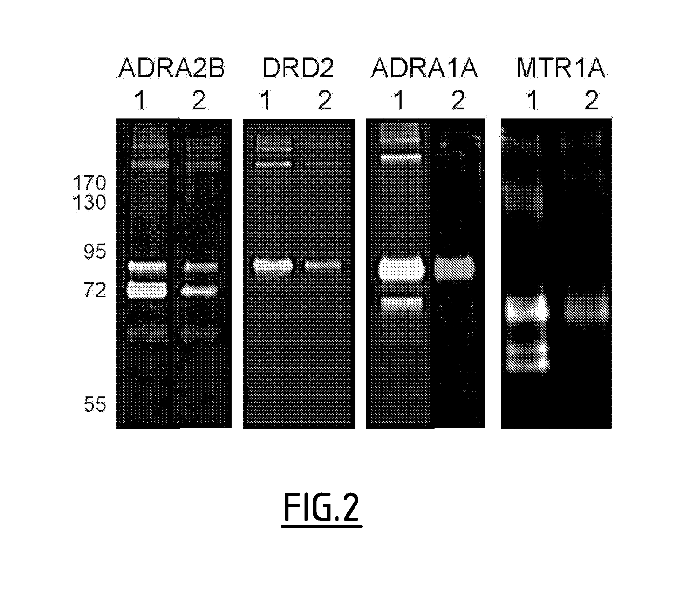

**FIG. 2**: Expression of recombinant GPCRs (ADRA2B, DRD2, ADRA1A, MTR1 A) in cell membrane fraction (crude membrane) (10 µg of membrane proteins) of wild type (lanes 1) and *erg6* mutant (lanes 2) *P. pastoris* cells by Western Blot with anti Flag M2 and IRDye antibodies. Molecular masses are indicated in kDa on the left.

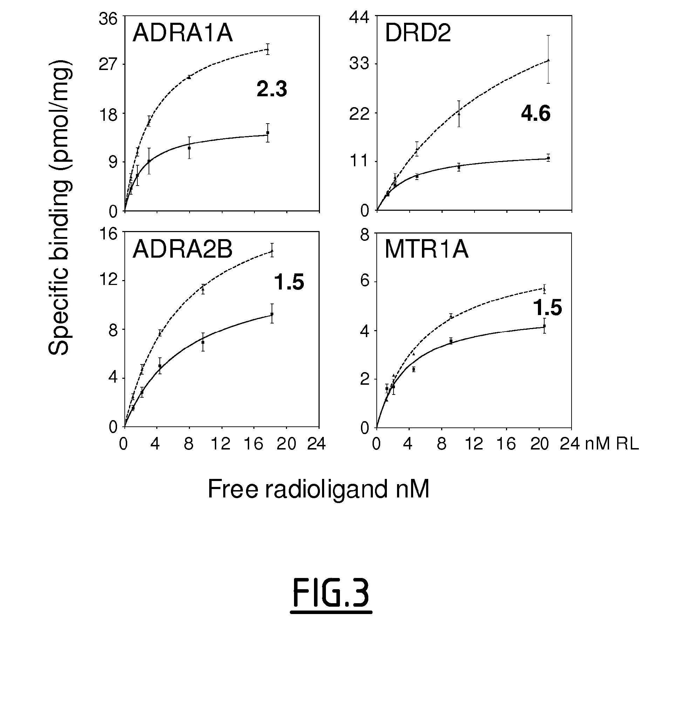

**FIG. 3**: Radioligand binding assay performed on cell membrane fraction prepared from wild type (continuous lines) and *erg6* mutant (dashed lines) *P. pastoris* cells producing ADRA1A, ADRA2B, DRD2 or MTR1A. The plots represent specific binding of radioligands to receptors. Data were fitted using the one site saturation binding model and data points represent the mean ± SE from at least three independent experiments performed in triplicates.

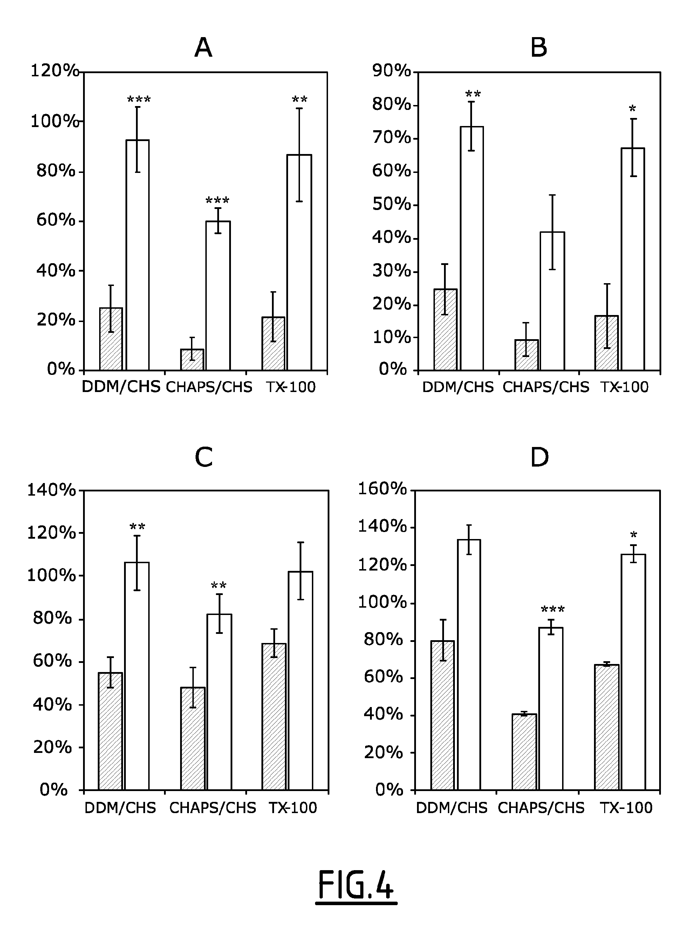

**FIG. 4**: Solubility of ADRA1A (A), ADRA2B (B), DRD2 (C) and MTR1A (D) after detergent extraction from cell membrane fraction (crude extract) from wild type (grey) or erg6 mutant (white) *P. pastoris* cells with DDM/CHS, CHAPS/CHS or Triton X-100 1 %. The amounts of receptor were quantified, setting total receptor in the membranes to 100 %. Results presented are the means ± standard deviation of at least three independant experiments, except where otherwise stated. Statistical analysis was undertaken using a two-tailed Student t test for comparison between means of two different groups. P < 0.05 (*) and P < 0.005 (**).

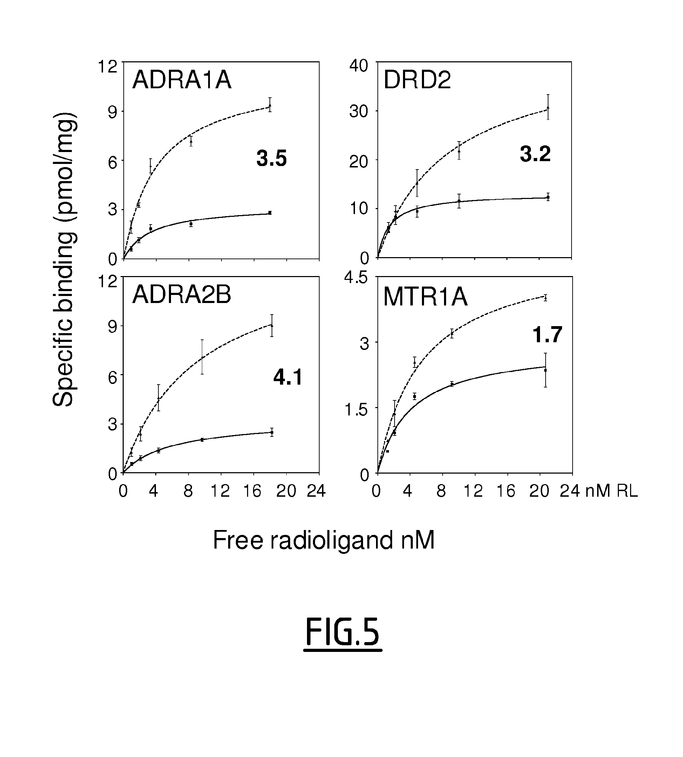

**FIG. 5**: Radioligand binding assay performed on purified recombinant_membrane protein fraction (solubilized receptors ADRA1A, ADRA2B, DRD2 or MTR1A ) prepared from membrane of wild type (continuous lines) and *erg6* mutant (dashed lines) *P. pastoris* cells producing ADRA1A, ADRA2B, DRD2 or MTR1A. The plots represent specific binding of radioligands to receptors. Data were fitted using the one site saturation binding model and data points represent the mean ± SE from at least three independent experiments performed in triplicates.

### EXAMPLES

### EXAMPLE 1: Preparation of mutated P. Pastoris yeast strains defective in membrane ergosterol by UV-irradiation

The study was realized on *P. pastoris* clones already carrying different recombinant receptors integrated in the yeast genome. The recombinant yeasts were obtained from the MePNet consortium (Lundstrom et al. 2006 J. Struct. Funct. Genomics. 7:77-91). Four different GPCRs were presently analyzed namely ADRA1A_Human, ADRA2B_Human, DRD2_Human and MTR1A_Human.
1) Recombinant *P. pastoris* GS115 cells were grown overnight on YPD plates (1% yeast extract, 2% peptone, 2% glucose, 2 % agar).
2) Yeast cells were exposed to a UV-transilluminator (254 nm, 10 sec).
3) UV-irradiated cells were allowed to grow on YPD plates supplemented with 10µg/mL nystatin for 3 days at 30°C.
4) Desired mutation events were selected by isolating nystatin-resistant colonies.
5) A second screen on 10 µg/mL nystatin was realized and susceptibility to 50 µg/mL cycloheximide, a common protein synthesis inhibitor, was also examined. In this context, after 3 days at 30°C, only mutants that were both nystatin-resistant and cycloheximide-sensitive were selected for further studies.
6) A PCR amplification of the *erg6* gene was carried out on the genomic DNA of the nystatin-resistant cells prepared by the phenol/chloroform extraction technique. 50 ng of genomic DNA were used as template for amplification using the forward primer oliE6 (SEQ ID N: 5) and the reverse primer oliantiE6 (SEQ ID N: 6). The amplification conditions were as follows: initial denaturation for 4 min at 94°C, then 30 cycles were carried out consisting of a denaturation step for 30 s at 94°C, a hybridization step for 30 s at 55°C, and then an elongation step for 30 s at 72°C. The reaction was terminated by a final extension for 5 min at 72 °C.
7) DNA sequencing of the PCR-amplified *erg6* gene from the different mutant strains revealed unique nonsense mutations resulting in truncated forms of the encoded Erg6 protein.

### EXAMPLE 2: Preparation of mutated P. Pastoris yeast strains defective in membrane ergosterol by directed mutagenesis of the erg6 gene

The study was realized using the same recombinant yeasts as in Example 1.

The directed mutagenesis of *ERG6* was carried out using a gene replacement method where the *ERG6* wild type gene was exchanged by a mutated erg6 gene following a homologous double recombination.
1) Production of mutated *erg6 gene*
   The mutated *erg6 gene* corresponded to a PCR amplified DNA fragment obtained from an erg6 mutant strain as described in Example1. In the present example, the nonsense mutation introducing a stop codon was located at position 363 of the gene (represented by SEQ ID N°2). The 1152 bp long PCR fragment was then electroeluted and purified and was directly used to transform *Pichia pastoris* cells.
2) Yeast transformation
   The same recombinant strains as in Example1 were transformed by electroporation with the PCR product carrying the nonsense mutation using a Gene Pulser I electroporator (Bio-Rad) and the following settings: 1500 V, 25 µF, and 600 Ω.
3) Selection of *erg6* mutant yeasts
   After transformation, the recombinant clones were selected for their resistance to nystatin 10 µg/mL and their susceptibility to cycloheximide 50 µg/mL.

### EXAMPLE 3: Sterol composition in membranes

With the aim of verifying that the ERG6 gene product is deficient in the nystatin-resistant strains, an analysis of the sterol compositions by gaz chromatography and by high pressure liquid chromatography was carried out as follows:
1) Sterol extraction
   Total sterols were directly extracted from crude membranes prepared from both WT and mutant strains for comparison.
   a) Membrane preparation
      - *P. Pastoris* cells were precultured in BMGY medium (1% yeast extract, 2 % peptone, 1.34 % yeast nitrogen base, 1 % glycerol, 0.1 M phosphate buffer, pH 6) overnight at 30°C and 250 rpm.
      - Induction was carried out in BMMY medium (1% yeast extract, 2 % peptone, 1.34 % yeast nitrogen base, 0.1 M phosphate buffer, pH 6, 0.5 % methanol) at 22 °C from an initial OD₆₀₀ₙₘ value of 5.
      - After a 18 h-induction, cells were pelleted (5000 rpm, 5 min), washed once in water and mecanically lysed in ice-cold TNE buffer (50 mM Tris-HCl, pH 7.4, 0.5 M NaCl, 2 mM DTT, 1 mM PMSF and 5 % glycerol) by vortexing vigorously with glass beads three times for 40 sec at 4 °C.
      - Then, the lysate was cleared of unbroken cells and debris by low-speed centrifugation (4000 rpm, 5 min, 4 °C).
      - Crude membranes were then pelleted by ultracentrifugation (45 000 rpm, 45 min, 4°C) and resuspended in TNE buffer. Proteins were quantified applying the Pierce BCA assay where BSA was used as the standard.
   b) Sterol extraction
      - 20 mg of membrane proteins were resuspended in 1.5 mL methanol, 1 mL 60 % potassium hydroxide, 1 mL 0.5 % pyrogallol and heated for 2 hours at 85 °C.
      - Total sterols were extracted twice with 3 mL cyclohexane (1/1, v/v).
      - The organic phase containing extracted sterols was dried under nitrogen and finally resuspended in 0.5 mL methanol.
2) Sterol separation
   Total sterols were separated by HPLC (Gilson HPLC chain with a UV detector set at 280 nm). The reverse phase measurements were carried out at RT on a 5 µm Gemini-NX C18 Phenomenex column (150 x 4.6 mm). The isocratic elution was done with HPLC grade methanol. The mobile phase flow rate was 1 mL/min and the injection volume was 10 µL. Ergosterol was also analyzed as a control.
   Chromatographic elution patterns of the sterol species revealed severe changes in the sterol composition of nystatin-resistant compared to WT cell membranes (**FIG. 1**). As expected, ergosterol which elutes with a retention time (RT) of 6.8 min is the sole sterol in WT crude membranes (**FIG. 1**). In contrast, ergosterol was undetectable in nystatin-resistant cell membranes. Instead, two new predominant specific sterol intermediates (RT of 5 and 5.9 min, **FIG. 1**) accumulated in the crude membranes of mutated cells.
3) Sterol characterization
   Finally, sterols were analyzed using GC/MS to identify major components.
   GC-MS analysis of sterols confirmed that mutant strains contained no ergosterol (MW: 396). In contrast, they contained sterols with molecular weights of 382 (cholesta-5,7,24 trienol, 5.9 min) and 380 (cholesta-5,7,22,24 tetraenol, 5 min).
   These analyses confirmed the absence of ergosterol synthesis and the accumulation of intermediates sterols compatible with the expected disruptions of the biosynthetic pathway in the mutant strains.

### EXAMPLE 4: Effect of ergosterol deficiency upon recombinant GPCR expression and ligand binding properties

To determine whether membrane ergosterol affect receptor expression and functionality, a comparative study was conducted using membranes from ERG6 (WT) and erg6 (mutant) strains expressing the same GPCRs. For the production of recombinant GPCRs, membranes were prepared as in Example 3 from the recombinant yeasts described in Example 1.

### 1) GPCR expression

Total receptor production in both *P. pastoris* membrane preparations was assessed by Western blot studies using the Flag-tag present on each receptor (**FIG. 2**). Protein fractions (10 µg) were loaded onto a 10 % polyacrylamide gel according to (Schagger and Von Jagow 1987 Anal Biochem. 166:368-79), and blotted onto a nitrocellulose membrane. For immunodetection, the nitrocellulose membrane was incubated with the mouse monoclonal anti-Flag M2 antibody (Sigma-Aldrich®) at 1:8,000. The detection of primary antibody was carried out using fluorescent IRDye 100cw donkey anti-mouse IgG (LI-COR Biosciences®) as secondary antibodies. Bands were visualized and quantified with an Odyssey Infrared Imaging System (LI-COR Bioscience®). For all four GPCRs, the more fluorescent band corresponds to the monomeric form. However, other upper bands corresponding to oligomeric forms (about 200 kDa and more) were observed particularly when the receptor was expressed in the WT *ERG6* strain. In ergosterol-deficient erg6 cells, these oligomeric forms were not observed as in the case of ADRA1A, DRD2 and MTR1A or were visible to a much lower extent for ADRA2B. This indicates that absence of ergosterol in *erg6* strains allows the expression of more homogeneous receptors, thus providing a more suitable environment. To quantify the relative signals, fluorescence intensity was monitored, and for one receptor, expression level in *erg6* cells was calculated as the percentage of the expression level of the same receptor in the *ERG6* WT cells. In all cases the absence of ergosterol resulted in a decrease in the expression of total receptors (from 2 fold to 4 fold) (**FIG. 2**).

### 2) GPCR functionality

Whether absence of ergosterol could affect the amount of active protein was checked by saturation experiments on both crude membrane preparations. For saturation binding assays, increasing concentrations of radioactive ligands ranging from 1 to 30 nM were added to 10 µg of proteins diluted in binding buffer (30 mM HEPES, pH 7.7, 0.15 M NaCl and 0.1% BSA). The radioactive ligands used were [3H]-Spiperone, [3H]-Prazozine, [3H]-Rauwolscine and [3H]-Melatonin for DRD2, ADRA1, ADR2B and MTR1A, respectively. Non-specific binding was determined by the addition of 10 µM « cold » ligands using spiperone, prazozine, yohimbine and melatonin for DRD2, ADRA1, ADR2B and MTR1A, respectively. After 1-h incubation at RT, the reaction was terminated by rapid filtration of the samples through GF/B filters presoaked in 1 % polyethylenimine. The filters were washed 3 times with ice-cold buffer (Tris-HCI 50 mM, pH 7.4), and the radioactivity was measured by liquid-scintillation counting. The equilibrium dissociation constant, K_{D} and the maximal number of binding sites, Bmax for radioligands were determined. The data were expressed as the mean ± standard error (SE) for at least three independant experiments performed in triplicate. Data were fitted through non-linear regression analysis with the one-site saturation binding model using the Prism 4 program of GraphPad. As shown in **FIG.3**, all four recombinant receptors displayed single class of specific and saturable binding sites allowing the determination of their respective Bmax and Kd values that are reported in **Table 1.**

**Table 1: Binding parameters of expressed receptors in membranes of WT and erg6 cells.**

| | | **ADRA1A** | **ADRA2B** | **DRD2** | **MTR1A** |
|---|---|---|---|---|---|
| **WT** | **Bmax (pmol/mg)** | 15.72±2.09 | 12.97±1.58 | 13.54±095 | 4.86±0.38 |
| | **K_{D}(nM)** | 2.21±0.93 | 7.61±2 09 | 3.44±0.73 | 3.62±0.83 |
| ***erg6*** | **Bmax(pmol/mg)** | 35.74±1.11 | 20.09±0.97 | 62.20±16.42 | 1.35±0.30 |
| | **K_{D} (nM)** | 3.52±0.30 | 7 20±0.80 | 17.69±8.24 | 5.86±0.61 |

Expression of the four tested receptors in WT *ERG6* cells yielded production levels (Bmax) ranging from 4.86 ± 0.38 (MTR1 A) to 15.72 ± 2.09 pmol bound ligand per mg total membrane protein (ADRA1A) and revealed high affinity binding for their radioligand (Kd) ranging from 2.21 ± 0.93 (ADRA1 A) to 7.61 ± 2.09 nM (ADRA2B). Interestingly, while Kd values were comparable irrespective of the sterol background of the host strain, Bmax values were about twofold higher in membranes lacking ergosterol compared to ergosterol-rich membranes.

Altogether, the results indicated that altering ergosterol content increased the amount of functional receptors, as assessed by ligand binding and decreased the overall amount of GPCRs proteins expressed, as assessed by Western blot experiments. Accordingly, lack of ergosterol resulted in a dramatic increase in the ratio of ligand binding competent receptors to total expressed receptors, therefore strongly suggesting of a much higher homogeneity of the expressed receptors.

### EXAMPLE 5: Effect of ergosterol deficiency upon GPCR solubilization and soluble receptor activity

To determine whether ergosterol affect the GPCR extraction from yeast membranes, a comparative study was conducted using membranes from ERG6 and erg6 strains expressing the same GPCRs.

### 1) GPCR solubilization

The same yeast membranes as in Example 4 were solubilized in the presence of different detergents (DDM/CHS, CHAPS/CHS, Triton-X100) at a final concentration of 1 %. Solubilization assays were realized at a protein concentration of 4 mg/mL at 4°C in a final volume of 500 µL. Detergent-insoluble material was removed by ultracentrifugation at 40 000 rpm for 10 min at 4 °C.

After detergent extraction, the relative amount of soluble receptors recovered in supernatants was analyzed by dot blot experiments following the immunodetection procedure described in Example 4. To analyze the solubilization efficiency, fluorescence intensity of the signals obtained after detergent treatment were quantified and compared to those obtained in crude membranes (whole receptor signal = 100 %) (**FIG. 4**). In *ERG6* cells, the four tested receptors exhibited two different behaviors upon solubilization. ADRA1 A and ADRA2B as members of the first category were very hardly extracted from membranes with any of the detergents tested. Detergents only extracted up to 20 % of receptors, suggesting that a great fraction of receptors (up to 80 %) are resistant to detergent treatment. DRD2 and MTR1A belonging to a second category were less resistant to detergent treatment. More than 60 % of total receptor were efficiently extracted using Triton X-100.

Remarkably, all the receptors were efficiently extracted from membranes of ergosterol-deficient erg6 cells (**FIG. 4**). Whatever the detergent used indeed, the amount of recovered receptor after extraction was systematically increased, with fractions close to 100 % for all the receptors when using Triton X-100.

### 2) GPCR functionality

Saturation binding experiments were performed on CHAPS/CHS solubilized receptors the same way than described in Example 4 in order to test receptor activity and stability (**FIG.5**). The respective pharmacological parameters deduced from these experiments are given in **Table 2**.

**Table 2: Binding parameters of solubilized receptors from membranes of WT and erg6 cells.**

| | | **ADRA1A** | **ADRA2B** | **DRD2** | **MTR1A** |
|---|---|---|---|---|---|
| **WT** | **Bmax (pmol/mg)** | 3.23±0 27 | 3.27±031 | 13 10±1 04 | 2.91±0.29 |
| | **K_{D}(nM)** | 3.2±0.78 | 5.84±1.41 | 1.48±0.48 | 4.06±1.14 |
| ***erg6*** | **Bmax(pmol/mg)** | 11.34±0.71 | 13.65±2.15 | 42.79±4.71 | 5.13±0.32 |
| | **K_{D}(nM)** | 4.08±0.68 | 9.16±3.05 | 8.77±2.14 | 5.47±0.88 |

Accordingly, while Kd values were comparable irrespective of the sterol background of the host strain, Bmax values were more than two fold higher for the receptors solubilized from membranes lacking ergosterol compared to those extracted from ergosterol-rich membranes.

Overall, the results indicate that the lack of ergosterol and thus the alteration of rafts allowed an improved susceptibility of the recombinant GPCRs receptors to the solubilizing action of the detergents and resulted in a marked increase in solubilized receptor activity and stability.

## Claims

1. A method for producing a recombinant GPCR protein in yeast which comprises:
- providing a yeast strain deficient in a raft lipid biosynthesis,
- transforming said yeast strain with an expression vector comprising a sequence coding for said recombinant GPCR protein, and
- growing said yeast strain under conditions allowing expression of said recombinant GPCR protein.

2. The method of claim 1, wherein said raft lipid is ergosterol.

3. A method according to claim 1 or 2, comprising a step of extracting, and optionally purifying, said recombinant GPCR protein from the yeast membrane.

4. A method according to any one of claims 1 to 3, wherein the yeast strain is a *Pichia pastoris* strain.

5. A method according to any one of claims 1 to 4, wherein the number of recombinant GPCR proteins in an active configuration compared to the total number of recombinant GPCR proteins is at least 10 % higher in the yeast strain deficient in raft lipid biosynthesis than it would be in yeast strains synthesizing said raft lipid.

6. A method according to any one of claims 2 to 5, wherein the recombinant GPCR protein is extracted from the yeast membrane by a detergent.

7. A method according to anyone of claims 1 to 6, wherein the recombinant GPCR protein which is produced carries a tag sequence selected in the group comprising a flag tag, an EGFP tag, a histidine tag and a biotinylation tag.

8. A method for screening for a ligand of a recombinant membrane protein comprising the steps of:
i) providing a yeast strain deficient in a raft lipid biosynthesis,
ii) transforming said yeast strain with an expression vector comprising a sequence coding for said recombinant membrane protein,
iii) growing said yeast strain under conditions allowing expression of said recombinant membrane protein,
iv) contacting at least one candidate ligand with whole cells of the yeast strain grown at step iii), with a cell membrane fraction obtained therefrom, with a solubilized membrane protein fraction obtained therefrom or with a purified recombinant membrane protein fraction obtained therefrom, and
v) determining whether said candidate compound binds to the recombinant membrane protein.

9. A method for crystallizing a recombinant membrane protein comprising:
i) providing a yeast strain deficient in a raft lipid biosynthesis,
ii) transforming said yeast strain with an expression vector comprising a sequence coding for said recombinant membrane protein,
iii) growing said yeast strain under conditions allowing expression of said recombinant membrane protein.
iv) extracting and purifying said recombinant membrane protein, and
v) crystallizing the recombinant membrane protein thus obtained.

10. A method for crystallizing a membrane protein according to claim 9, wherein said method comprises the step of stabilizing the recombinant membrane protein by adding a ligand of a recombinant membrane protein before the step of extracting and purifying said membrane protein.

11. A method for crystallizing a recombinant membrane protein according to claim 9 or 10, wherein the recombinant membrane protein is crystallized in an active form.

12. Use of a yeast strain deficient in a raft lipid biosynthesis for the production of a recombinant GPCR protein.

13. A yeast strain deficient in a raft lipid biosynthesis transformed with an expression vector comprising the sequence coding for a recombinant GPCR protein.

14. A kit for producing a recombinant membrane protein comprising:
― a yeast strain deficient in a raft lipid biosynthesis;
― buffers and/or reagents suitable for producing a recombinant membrane protein; and
― instructions for use in the production of a recombinant membrane protein.

15. The method according to any one of claims 8 to 11, the use according to claim 12, the yeast strain according to claim 13 or the kit according to claim 14, wherein said raft lipid is ergosterol.


SEQUENCE LISTING

<110>  INSERM

<120>  A raft deficient yeast expression system for the production of
       recombinant membrane proteins

<130>  BET10P0968

<160>  6

<170>  PatentIn version 3.4

<210>  1
<211>  1152
<212>  DNA
<213>  Pichia pastoris


<220>
<221>  CDS
<222>  (1)..(1152)

<400>  1
atg act acc tct aca act gaa caa gaa agc cag cca ctt gcc cca aag        48
Met Thr Thr Ser Thr Thr Glu Gln Glu Ser Gln Pro Leu Ala Pro Lys
1               5                   10                  15

aac tat gag caa gat aga gaa ttt gca aag gct ttg cac ggc aag gat        96
Asn Tyr Glu Gln Asp Arg Glu Phe Ala Lys Ala Leu His Gly Lys Asp
            20                  25                  30

gcc gct aag aaa acc ggt ctc gga gct tgg gtc aca aag gac gcc tca       144
Ala Ala Lys Lys Thr Gly Leu Gly Ala Trp Val Thr Lys Asp Ala Ser
        35                  40                  45

gcc caa gaa gta gcg gtc gat ggc tac ttc aag cac tgg aat ggt aag       192
Ala Gln Glu Val Ala Val Asp Gly Tyr Phe Lys His Trp Asn Gly Lys
    50                  55                  60

act gat cag gaa gct gaa cac tca aga aag agt gat tat tcg gag ctg       240
Thr Asp Gln Glu Ala Glu His Ser Arg Lys Ser Asp Tyr Ser Glu Leu
65                  70                  75                  80

acc aag cat tac tac aac ctg gtt act gat ttc tac gag tac ggt tgg       288
Thr Lys His Tyr Tyr Asn Leu Val Thr Asp Phe Tyr Glu Tyr Gly Trp
                85                  90                  95

ggt tct tcg ttt cat ttt tct aga tac tac aga gga gag gcc ttc aga       336
Gly Ser Ser Phe His Phe Ser Arg Tyr Tyr Arg Gly Glu Ala Phe Arg
            100                 105                 110

cag gct acc gct agg cac gaa cat tac ctg gcc ctg aaa atg ggt atc       384
Gln Ala Thr Ala Arg His Glu His Tyr Leu Ala Leu Lys Met Gly Ile
        115                 120                 125

acc gaa aac atg aaa gtt ttg gac gtt gga tgt ggt gtc ggt ggt cca       432
Thr Glu Asn Met Lys Val Leu Asp Val Gly Cys Gly Val Gly Gly Pro
    130                 135                 140

gca aga gag att gcc cgt ttc act ggt tgt tct gtc gtt ggt tta aac       480
Ala Arg Glu Ile Ala Arg Phe Thr Gly Cys Ser Val Val Gly Leu Asn
145                 150                 155                 160

aac aac gat tac cag gtt gaa aga gca gaa ttc tat tcc aag aaa tac       528
Asn Asn Asp Tyr Gln Val Glu Arg Ala Glu Phe Tyr Ser Lys Lys Tyr
                165                 170                 175

aac atg acg aag caa ttg tca tac gta aaa ggt gac ttc atg caa atg       576

Asn Met Thr Lys Gln Leu Ser Tyr Val Lys Gly Asp Phe Met Gln Met
            180                 185                 190

gac ttt gag ccc gag acc ttc gat gct gtg tac gcc att gag gcc act      624
Asp Phe Glu Pro Glu Thr Phe Asp Ala Val Tyr Ala Ile Glu Ala Thr
        195                 200                 205

gtt cac gcc cca gtg ttg gaa ggt gtt tat agt gag att tac aag gtg      672
Val His Ala Pro Val Leu Glu Gly Val Tyr Ser Glu Ile Tyr Lys Val
    210                 215                 220

ctg aaa cca gga gga gca ttt ggg gtg tat gag tgg gtt atg acc gat      720
Leu Lys Pro Gly Gly Ala Phe Gly Val Tyr Glu Trp Val Met Thr Asp
225                 230                 235                 240

gca tat gac gaa aca aac ccg gaa cac cgt gcc att gcc tac ggt att      768
Ala Tyr Asp Glu Thr Asn Pro Glu His Arg Ala Ile Ala Tyr Gly Ile
                245                 250                 255

gaa gtt ggt gac ggt atc cca aag atg tac aag aga caa gtt gca gaa      816
Glu Val Gly Asp Gly Ile Pro Lys Met Tyr Lys Arg Gln Val Ala Glu
            260                 265                 270

gat gca ctg aaa aat gtc gga ttt gaa gta gag tac cag aaa gac atg      864
Asp Ala Leu Lys Asn Val Gly Phe Glu Val Glu Tyr Gln Lys Asp Met
        275                 280                 285

gca gac atg gac gac gat atc cca tgg tac tat cct ctt tcc ggt caa      912
Ala Asp Met Asp Asp Asp Ile Pro Trp Tyr Tyr Pro Leu Ser Gly Gln
    290                 295                 300

tta aag tat gtc cag act ctg ggt gac ata ttc acc atc ttc aga act      960
Leu Lys Tyr Val Gln Thr Leu Gly Asp Ile Phe Thr Ile Phe Arg Thr
305                 310                 315                 320

agt aag att ggt aga aca gtt acc act gaa acc gtt gga tta atg gaa     1008
Ser Lys Ile Gly Arg Thr Val Thr Thr Glu Thr Val Gly Leu Met Glu
                325                 330                 335

aag ttg gga ttg gcc ccc aag ggt tca aga caa gtt acc aac gca ttg     1056
Lys Leu Gly Leu Ala Pro Lys Gly Ser Arg Gln Val Thr Asn Ala Leu
            340                 345                 350

gaa gat gcc gcc gtt aac ttg gtg aag ggt ggt gaa ctt aag ctc ttt     1104
Glu Asp Ala Ala Val Asn Leu Val Lys Gly Gly Glu Leu Lys Leu Phe
        355                 360                 365

aca ccc atg atg tta tac gtt gcc aga aaa cca ttg gat gcc aaa taa     1152
Thr Pro Met Met Leu Tyr Val Ala Arg Lys Pro Leu Asp Ala Lys
    370                 375                 380

<210>  2
<211>  383
<212>  PRT
<213>  Pichia pastoris

<400>  2

Met Thr Thr Ser Thr Thr Glu Gln Glu Ser Gln Pro Leu Ala Pro Lys
1               5                   10                  15

Asn Tyr Glu Gln Asp Arg Glu Phe Ala Lys Ala Leu His Gly Lys Asp
            20                  25                  30

Ala Ala Lys Lys Thr Gly Leu Gly Ala Trp Val Thr Lys Asp Ala Ser
        35                  40                  45

Ala Gln Glu Val Ala Val Asp Gly Tyr Phe Lys His Trp Asn Gly Lys
    50                  55                  60

Thr Asp Gln Glu Ala Glu His Ser Arg Lys Ser Asp Tyr Ser Glu Leu
65                  70                  75                  80

Thr Lys His Tyr Tyr Asn Leu Val Thr Asp Phe Tyr Glu Tyr Gly Trp
                85                  90                  95

Gly Ser Ser Phe His Phe Ser Arg Tyr Tyr Arg Gly Glu Ala Phe Arg
            100                 105                 110

Gln Ala Thr Ala Arg His Glu His Tyr Leu Ala Leu Lys Met Gly Ile
        115                 120                 125

Thr Glu Asn Met Lys Val Leu Asp Val Gly Cys Gly Val Gly Gly Pro
    130                 135                 140

Ala Arg Glu Ile Ala Arg Phe Thr Gly Cys Ser Val Val Gly Leu Asn
145                 150                 155                 160

Asn Asn Asp Tyr Gln Val Glu Arg Ala Glu Phe Tyr Ser Lys Lys Tyr
                165                 170                 175

Asn Met Thr Lys Gln Leu Ser Tyr Val Lys Gly Asp Phe Met Gln Met
            180                 185                 190

Asp Phe Glu Pro Glu Thr Phe Asp Ala Val Tyr Ala Ile Glu Ala Thr
        195                 200                 205

Val His Ala Pro Val Leu Glu Gly Val Tyr Ser Glu Ile Tyr Lys Val
    210                 215                 220

Leu Lys Pro Gly Gly Ala Phe Gly Val Tyr Glu Trp Val Met Thr Asp
225                 230                 235                 240

Ala Tyr Asp Glu Thr Asn Pro Glu His Arg Ala Ile Ala Tyr Gly Ile
                245                 250                 255

Glu Val Gly Asp Gly Ile Pro Lys Met Tyr Lys Arg Gln Val Ala Glu
            260                 265                 270

Asp Ala Leu Lys Asn Val Gly Phe Glu Val Glu Tyr Gln Lys Asp Met
        275                 280                 285

Ala Asp Met Asp Asp Asp Ile Pro Trp Tyr Tyr Pro Leu Ser Gly Gln
    290                 295                 300

Leu Lys Tyr Val Gln Thr Leu Gly Asp Ile Phe Thr Ile Phe Arg Thr
305                 310                 315                 320

Ser Lys Ile Gly Arg Thr Val Thr Thr Glu Thr Val Gly Leu Met Glu
                325                 330                 335

Lys Leu Gly Leu Ala Pro Lys Gly Ser Arg Gln Val Thr Asn Ala Leu
            340                 345                 350

Glu Asp Ala Ala Val Asn Leu Val Lys Gly Gly Glu Leu Lys Leu Phe
        355                 360                 365

Thr Pro Met Met Leu Tyr Val Ala Arg Lys Pro Leu Asp Ala Lys
    370                 375                 380

<210>  3
<211>  1152
<212>  DNA
<213>  artificial sequence

<220>
<223>  erg6 mutant gene

<220>
<221>  CDS
<222>  (1)..(363)

<400>  3
atg act acc tct aca act gaa caa gaa agc cag cca ctt gcc cca aag       48
Met Thr Thr Ser Thr Thr Glu Gln Glu Ser Gln Pro Leu Ala Pro Lys
1               5                   10                  15

aac tat gag caa gat aga gaa ttt gca aag gct ttg cac ggc aag gat       96
Asn Tyr Glu Gln Asp Arg Glu Phe Ala Lys Ala Leu His Gly Lys Asp
            20                  25                  30

gcc gct aag aaa acc ggt ctc gga gct tgg gtc aca aag gac gcc tca      144
Ala Ala Lys Lys Thr Gly Leu Gly Ala Trp Val Thr Lys Asp Ala Ser
        35                  40                  45

gcc caa gaa gta gcg gtc gat ggc tac ttc aag cac tgg aat ggt aag      192
Ala Gln Glu Val Ala Val Asp Gly Tyr Phe Lys His Trp Asn Gly Lys
    50                  55                  60

act gat cag gaa gct gaa cac tca aga aag agt gat tat tcg gag ctg      240
Thr Asp Gln Glu Ala Glu His Ser Arg Lys Ser Asp Tyr Ser Glu Leu
65                  70                  75                  80

acc aag cat tac tac aac ctg gtt act gat ttc tac gag tac ggt tgg      288
Thr Lys His Tyr Tyr Asn Leu Val Thr Asp Phe Tyr Glu Tyr Gly Trp
                85                  90                  95

ggt tct tcg ttt cat ttt tct aga tac tac aga gga gag gcc ttc aga      336
Gly Ser Ser Phe His Phe Ser Arg Tyr Tyr Arg Gly Glu Ala Phe Arg
            100                 105                 110

cag gct acc gct agg cac gaa cat taa ctggccctga aaatgggtat            383
Gln Ala Thr Ala Arg His Glu His
        115                 120

caccgaaaac atgaaagttt tggacgttgg atgtggtgtc ggtggtccag caagagagat    443

tgcccgtttc actggttgtt ctgtcgttgg tttaaacaac aacgattacc aggttgaaag    503

agcagaattc tattccaaga aatacaacat gacgaagcaa ttgtcatacg taaaaggtga    563

cttcatgcaa atggactttg agcccgagac cttcgatgct gtgtacgcca ttgaggccac 623

tgttcacgcc ccagtgttgg aaggtgttta tagtgagatt tacaaggtgc tgaaaccagg 683

aggagcattt ggggtgtatg agtgggttat gaccgatgca tatgacgaaa caaacccgga 743

acaccgtgcc attgcctacg gtattgaagt tggtgacggt atcccaaaga tgtacaagag 803

acaagttgca gaagatgcac tgaaaaatgt cggatttgaa gtagagtacc agaaagacat 863

ggcagacatg gacgacgata tcccatggta ctatcctctt tccggtcaat taaagtatgt 923

ccagactctg ggtgacatat tcaccatctt cagaactagt aagattggta gaacagttac 983

cactgaaacc gttggattaa tggaaaagtt gggattggcc cccaagggtt caagacaagt 1043

taccaacgca ttggaagatg ccgccgttaa cttggtgaag ggtggtgaac ttaagctctt 1103

tacacccatg atgttatacg ttgccagaaa accattggat gccaaataa 1152

<210> 4
<211> 120
<212> PRT
<213> artificial sequence

<220>
<223> Synthetic Construct

<400> 4

Met Thr Thr Ser Thr Thr Glu Gln Glu Ser Gln Pro Leu Ala Pro Lys
1               5                   10                  15

Asn Tyr Glu Gln Asp Arg Glu Phe Ala Lys Ala Leu His Gly Lys Asp
            20                  25                  30

Ala Ala Lys Lys Thr Gly Leu Gly Ala Trp Val Thr Lys Asp Ala Ser
        35                  40                  45

Ala Gln Glu Val Ala Val Asp Gly Tyr Phe Lys His Trp Asn Gly Lys
    50                  55                  60

Thr Asp Gln Glu Ala Glu His Ser Arg Lys Ser Asp Tyr Ser Glu Leu
65                  70                  75                  80

Thr Lys His Tyr Tyr Asn Leu Val Thr Asp Phe Tyr Glu Tyr Gly Trp
                85                  90                  95

Gly Ser Ser Phe His Phe Ser Arg Tyr Tyr Arg Gly Glu Ala Phe Arg
            100                 105                 110

Gln Ala Thr Ala Arg His Glu His
        115                 120

<210> 5
<211> 21
<212> DNA
<213> artificial sequence

<220>

<223>  primer

<400>  5
atgactacct ctacaactga a                                                21


<210>  6
<211>  20
<212>  DNA
<213>  artificial sequence

<220>
<223>  primer

<400>  6
ttatttggca tccaatggtt                                                  20